Europäisches Patentamt

European Patent Office

Office européen des brevets

(11) Veröffentlichungsnummer: **0 182 929**
A1

## (12) EUROPÄISCHE PATENTANMELDUNG

(21) Anmeldenummer: 84114247.4

(22) Anmeldetag: 26.11.84

(51) Int. Cl.⁴: **C 12 Q 1/56**
C 12 Q 1/38, G 01 N 33/86
C 12 Q 1/44, A 61 K 35/50

(43) Veröffentlichungstag der Anmeldung:
04.06.86 Patentblatt 86/23

(84) Benannte Vertragsstaaten:
AT BE CH DE FR GB IT LI LU NL SE

(71) Anmelder: AMERICAN HOSPITAL SUPPLY
CORPORATION
One American Plaza
Evanston, IL 60201(US)

(72) Erfinder:
Die Erfinder haben auf ihre Nennung verzichtet

(74) Vertreter: Brose, D. Karl, Dipl.-Ing. et al,
Patentanwälte Brose & Partner Wiener Strasse 2
D-8023 München-Pullach(DE)

(54) Koaktivator zur Aktivierung von Protein C.

(57) Es wird ein Koaktivator beschrieben, mit dem die Bestimmung von Protein C in menschlichem Plasma schnell und einfach durchführbar ist.

Koaktivator zur Aktivierung von Protein C

Die Erfindung bezieht sich auf einen Koaktivator zur Aktivierung von Human-Protein C in Gegenwart von Calciumionen.

Die Haemostase wird durch zwei Hauptmechanismen kontrolliert, nämlich einerseits die Gerinnung, welche das Entstehen eines Gerinnsels reguliert, und andererseits durch die Fibrinolyse, welche für die Regulation des Gerinnselabbaus verantwortlich ist.

Die Gerinnung verläuft nach einer Kaskadenreaktion, bei der durch sukzessive Aktivierung (Teilproteolyse) von im Blut zirkulierenden Gerinnungsfaktoren (Proteine) Thrombin gebildet wird, welches Fibrinogen in unlösliches Fibrin umwandelt.

Von den bekannten Mechanismen zur physiologischen Regulierung der Gerinnung kommt den beiden folgenden besondere Bedeutung zu:

i)    Durch Antithrombin III wird der Thrombin-Heparin-Komplex an die Oberfläche der Endothelialzellen gebunden, wodurch das Thrombin inhibiert und damit die Gerinnselbildung verhindert wird.

ii)   Protein C wird durch Thrombin zu aktiviertem Protein C (PCa) übergeführt, das als Serinprotease wirkt, welche die für die Gerinnung erforderlichen Faktoren V und VIII in aktivierter Form (Va und VIIIa) abbaut, wodurch die Gerinnselbildung inhibiert oder verzögert wird.

0182929

Protein C ist ein Vitamin K abhängiges Plasmaprotein mit einem Molekulargewicht von 62 000. Zum Aufbau und zur Funktion von Protein C ist auf folgende Literaturstellen hinzuweisen: Kisiel, W.: J. Clin. Invest. 64, 761-769 (1979); Sala, N., Owen, W. G., Collen, B.: Blood 63, 671-675 (1984); Cazenave, J. P., Wiesel, M.-L., Grunebaum, L., Freyssinet, J.-M., Dorner, M., Fritsché, R., Greber, S., Spaethe, R., Lampart, A.: VIIIth International Congress on Thrombosis, Istanbul (1984); Freyssinet, J.M., Grunebaum, L., Wiesel, M. L., Fritsché, R., Greber, S., Lampart, A.: Titisee Meeting on Protein C (1984); Esmon, C. T., Esmon, N. L.: Semin. Thromb. Hemostas. 10, 122-130 (1984); Salem, H. H. Broze, G. J., Miletich, J. P., Magerus, P. W.: Proc. Natl. Acad. Sci. USA 80, 1584-1588 (1983); Griffin, J. H.: Semin. Thromb. Hemstas. 10, 162-166 (1984); Bertina, R. M., Broeckmans, A. W., Krommenhoeck-van, E., van Wijngarden, A.: Thromb. Haemostas. 51, 1-5 (1984); Walker, F. J.: Thrombosis Research 22, 321-27 (1981).

Es wurde festgestellt, daß zur Umwandlung von Protein C zu aktiviertem Protein C, durch Thrombin in Gegenwart von Calciumionen in physiologischen Konzentrationen, Koaktivatoren erforderlich sind. Bisher sind zwei Koaktivatoren bekannt, und zwar sogenanntes Thrombomodulin, das aus Kaninchen - oder Rattenlunge gewonnen wird (Esmon, Charles T. Contemporary Hematology/Oncology, 1984, Seiten 137 - 154), sowie der Faktor Va (Salem, H. H. Broze, G. J., Miletich, J. P., Magerus, P. W.: Proc. Natl. Acad. Sci. USA 80, 1584-1588, 1983).

Weiterhin haben klinische Studien gezeigt, daß Familien mit häufigen Thrombosefällen einen angeborenen Mangel an Protein C aufweisen können (Griffin, H. H.: Semin. Thromb. Hemstas, 10, 162 - 166, 1984). Der Protein C-Gehalt im Plasma scheint neben der Thrombosediagnostik jedoch auch in anderen klinischen Fällen wichtig zu sein, z. B. für die disseminierte intravaskuläre Gerinnung.

Es ist bekannt, den Protein C-Gehalt im menschlichen Plasma durch die Laurell-Rocket-Immunelektrophorese mit einem Anti-Protein C-Antiserum zu ermitteln (Esmon, Charles T., Contemporary Hematology/Oncology, 1984, Seite 137 ff.). Dabei kann jedoch nur der Gesamtgehalt des Protein C ermittelt werden, nicht jedoch dessen biologische Aktivität, auf die es bei der Diagnose aber vor allem ankommt.

Auch sind bereits Funktionstests für Human-Protein C bekannt, also Methoden zur Bestimmung der biologischen Aktivität von Protein C (Sala, N., Owen, W.G., Broeckmanns, A. W., Krommenhoeck-van, E., van Wijngarden, A.: Thromb. Haemostas. 51, 1-5; 1984).

Diese Tests benötigen aber große Mengen an kostspieligen Reagenzien (humanes $\alpha$-Thrombin, Antithrombin III, Heparin, Hirudin, hochgereinigtes Kaninchen Thrombomodulin) und sind nur in einer Gesamtdauer von mindestens 6 Stunden durchführbar.

Es besteht daher ein großes Bedürfnis nach einer Bestimmungsmethode, insbesondere einem Funktionstest für Protein C in menschlichem Plasma, der mit routinemäßiger Analyse schnell, genau und mit geringem Kostenaufwand durchführbar ist.

Durch die Erfindung wird ein Koaktivator zur Aktivierung von Human-Protein C in Gegenwart von Calciumionen bereitgestellt, der im wesentlichen nach den im Anspruch 1 angegebenen Verfahrensschritten erhältlich ist.

Dieser Koaktivator (Extrakt) ist billig und ermöglicht die Durchführung des Funktionstests in ungefähr 2 Stunden. Die zusätzlichen Reagenzien ($\alpha$-Thrombin, Hirudin) werden außerdem in kleinen Mengen eingesetzt.

Es wurde festgestellt, daß der erfindungsgemäße Koaktivator einen Glyco-Proto-Lipid-Komplex der Zellmembran mit einem Molekulargewicht von 40 bis 100 000 Daltons darstellt.

Wird der erfindungsgemäße Koaktivator einer menschlichen Plasmaprobe zugegeben, so wird das Protein C rasch in aktiviertes Protein C umgewandelt. Das aktivierte Protein C (PCa) kann dann mit einem chromogenen Substrat-Verfahren bestimmt werden.

Durch den erfindungsgemäßen Koaktivator wird damit die Möglichkeit geschaffen, einen Protein C-Funktionstest als einfachen Routinetest durchzuführen.

Auch kann dieser Routinetest problemlos automatisiert werden. Als frisches menschliches Organ kommt in erster Linie die Placenta in Frage. Daneben ist auch die Verwendung von Lunge, Gehirn, Nieren und Leber denkbar. Ebenso sind Zellkulturen, insbesondere endotheliale Zellkulturen als Ausgangsmaterial zur Herstellung des erfindungsgemäßen Kofaktors geeignet.

Der erfindungsgemäße Funktionstest für Protein C im menschlichen Plasma kann qualitativ, halbqualitativ, quantitativ oder nach einem Screening-Verfahren durchgeführt werden. Statt einem chromogenen Substrat kann auch ein fluorogenes oder lumineszentes Substrat zur Bestimmung der amidolytischen Aktivität des aktivierten Protein C verwendet werden.

Neben der Bestimmung des Protein C-Gehalts aufgrund der amidolytischen Aktivität kann der Funktionstest auch nach einer Gerinnungsmethode durchgeführt werden. Das heißt, der Protein C-Gehalt wird anhand der Zeitdifferenz zwischen der aktivierten partiellen Thromoplastinzeit mit und ohne Koaktivator ermittelt.

Die nachstehenden Beispiele dienen der weiteren Erläuterung der Erfindung.

Beispiel 1

Dieses Beispiel bezieht sich auf die Extraktion des erfindungsgemäßen Koaktivators aus Placenta.

Frische Humanplacenta wird zerkleinert und mehrfach mit physiologischer Kochsalzlösung (0,9 Gew.-%) gewaschen. Danach wird einmal mit TRIS-HCl-Puffer (0,02 - 0,15 M), der Saccharose (0,05 - 0,3 M), Benzamidin (0,1 - 10 mM), Phenyl-methylsulfonyl-fluorid (0,1 - 2 mM) und NaN$_3$ (0,01 - 0,02 Gew.-%) enthält, gewaschen. Das Material wird anschließend auf -50 bis -80 °C tiefgefroren. Das aufgetaute Material wird homogenisiert und mit dem gleichen TRIS-Puffer gewaschen und anschließend bei 20 000 g 30 min bei 4 °C zentrifugiert. Das Präzipitat wird anschließend 1 bis 18 h bei pH 7,0 bis 8,0 mit einem TRIS-HCl-Puffer (20 bis 150 mM) der Saccharose (0,05 bis 0,3 M), Benzamidin (0,1 bis 10 mM), NaN$_3$ (0,01 bis 0,02 Gew.-%) und ein Detergenz (0,01 - 2 Gew.-%)

enthält, extrahiert. Als Detergenz können Triton X-100, LAPAO (Lauryl-amido-propyl-N,N-dimethylamin-oxid), Tween 20, 40, 60 oder 80, Gallensäuren (Cholate), Alkyl (n = 5-15)- Glyco- (oder Manno)-Pyranoside, NP 40, LUBROL PX (Ethylenoxid-Kondensat von Fettalkoholen), CHAPS (3-(3-Cholamidopropyl) dimethylaminio-3-propansulfat) oder STEROX (ethoxylierter Thioether) verwendet werden. Nach dem Zentrifugieren bei 30 000 g 30 min. bei 4 °C wird der Überstand mit mehreren Filtern (z. B. 3 µm, 1,2 µm, 0,8 µm und 0,4 µm) filtriert und anschließend konzentriert. Das Filtrat wird anschließend mit Phospholiphase $A_2$ (0,001 Einheiten/ml - 3 Einheiten /ml) aus Säugetier-Pancrease 10 min bis 10 h in Gegenwart von Calcium (5 mM) bei 22 bis 37 °C behandelt.

Der so gewonne Placenta-Extrakt wird durch Chromatographie mit DEAE-Sephacel weiter gereinigt. Der Protein C-Koaktivator wird dadurch von einer kontaminierenden amidolytischen Aktivität befreit, d. h. einer Aktivität, die den Funktionstest anhand der amidolytischen Aktivität beeinträchtigen würde. Eine Analyse auf SDS-PAGE des gereinigten Materials zeigte ein Protein-Band mit einer Verteilung zwischen 40 000 und 100 000 Daltons.

Beispiel 2

In diesem Beispiel wird die Extraktion des Protein C-Koaktivators unter spezifischer Verwendung von Triton X-100 beschrieben.

100 g frische Humanplazenta wurden zerkleinert und fünfmal mit einem Liter einer 0,9-gewichtsprozentigen wässrigen Nacl-Lösung in Gegenwart von 1 mM Phenylmethylsulfonylfluorid gewaschen. Anschließend wurde mit 2 Liter TRIS-HCl-Puffer (20 mM, pH 7,5), der 0,25 M Saccharose, 1 mM Benzamidin, 1 mM Phenylmethylsulfonylfluorid und 0,01 % (G/V) $NaN_3$ enthielt, gewaschen. Das Material wurde tiefgefroren und bei minus 70°C aufbewahrt.

Nach dem Auftauen wurde das Material mit einem elektrischen Gerät 3 Minuten lang homogenisiert. Danach wurde 1 Liter des oben erwähnten TRIS-HCl-Puffers hinzugegeben und anschließend fünfmal mit einem Liter dieses Puffers bei Raumtemperatur und unter Rühren gewaschen. Das Homogenat wurde mit 20000 g bei 4°C 30 Minuten lang zentrifugiert. Der Niederschlag wurde dann mit 30 ml TRIS-HCl-Puffer (20 mM , pH 7,5), der 0,25 M Saccharose, 1 mM Benzamidin, 0,01 % (G/V) $NaN_3$ und 0,5 (V/V) Triton X-100 enthielt, extrahiert. Diese Extraktion wurde 2 Stunden lang durchgeführt. Nach dem Zentrifugieren mit 30000 g bei 4°C 30 Minuten lang wurde der Überstand nacheinander durch Millipore-Membranen mit 3 μm, 1,2 μm, 4 μm, 1,2 μm, 0,8 μm und 0,4 μm filtriert und schließlich fünfmal mit einem Amicon-Gerät unter Verwendung von PM-10 Membranen konzentriert. Das Filtrat wird wie im Beispiel 1 mit Phospholipase $A_2$ behandelt.

## Beispiel 3

Dieses Beispiel beschreibt die Extraktion des Protein C-Koaktivators unter spezifischer Verwendung von Beta-D-octyl-glycosid.

Es wurde so vorgegangen wie im Beispiel 2, jedoch anstelle von Triton-X-100, Beta-D-octyl-glycosid mit einer Endkonzentration von 1 % (Gew/V) verwendet. Dieses Detergenz kann aus einem Gemisch nach der Extraktion leicht entfernt werden.

## Beispiel 4

Nach diesem Beispiel wird die biologische Aktivität des Koaktivators bestimmt, und zwar anhand dessen Fähigkeit, die Aktivierung von Protein C zu aktiviertem Protein C (PCa) durch Thrombin in Gegenwart von Calcium zu stimulieren.

Die Aktivierung wird in einem TRIS-HCl-Puffer (0,05 M, pH 7,5), der 0,1 M NaCl, 0,01 Gew.-% $NaN_3$, 5 mM $CaCl_2$ und 50 µg/ml Human-Albumin enthält, durchgeführt. Die Reagenz-Mischung enthält dabei bei einem Gesamtvolumen von 200 µl 1 bis 5 µg/ml Thrombin, 50 µg/ml Protein C und verdünnten Koaktivator und wird 30 min bei 37 °C inkubiert. Die Reaktion wird mit einem Überschuß an Thrombin-Inhibitor (beispielsweise Hirudin, $\alpha$-NAPAP, Antithrombin III und Heparin) gestoppt. Die Menge an aktiviertem Protein C (PCa) wird photometrisch, beispielsweise nach einem chromogenen, fluorogenen oder luminiphoren Substrattest gemessen. (z. B. Spaltet PCa das Chromogen-Substrat S 2238 und der somit freigesetzte para-Nitroanilin-Chromophor kann bei 405 nm photometrisch bestimmt werden).

## Beispiel 5

In diesem Beispiel wird ein Funktionstest zur Bestimmung der biologischen Aktivität des Protein C beschrieben. Dieser Test basiert auf der Fähigkeit des Koaktivators, die Umwandlung von Protein C zu aktiviertem PC (PCa) durch Thrombin in Gegenwart von Calcium zu stimulieren.

Der Test wird folgendermaßen durchgeführt:

1 ml Plasma (Probe, deren Protein C-Gehalt bestimmt werden soll)

+ 60 µl BaCl$_2$ 1 M

werden 20 min bei 4°C gerührt und anschließend zentrifugiert. Der Niederschlag wird zweimal mit 1 ml Imidazol-(20 mM) Puffer, pH 6,0, der Benzamidine (10 mM) und NaCl (0,2 M) enthält, gewaschen. Der gewaschene Niederschlag wird anschließend in 0,5 ml MES (2-N-Morpholino-ethane-sulfonic-acid) Puffer, pH 6,0, der 0,23 M Na-Citrat enthält, wieder aufgelöst. Durch Zugabe von Poly-ethylen-glycol werden alle Proteine, und dadurch auch Protein C, wieder präzipitiert. Nach Zentrifugation wird der Niederschlag in 200 µl TRIS (50 mM)-NaCl (0,1 M)-Puffer, pH 7,5, resupendiert und CaCl$_2$ (5 mM), Thrombin (0,5 Einheiten) sowie 50 µl Koaktivatorlösung werden dazugemischt (Totalvolumen 300 µl). Das Gemisch wird 30 min bei 37°C inkubiert und die Reaktion danach mit 5 Einheiten (ATU) Hirudin gestoppt.

Das dermaßen aktivierte PC (PCa) wird durch Zusatz eines chromogenen Substrats photometrisch oder durch die APTT-Verlängerung (APTT = aktivierte partielle Thromboplastinzeit) gemessen.

a) Chromogenische Substrat Messung

    300 µl der PCa-Lösung
+ 650 µl des oben erwähnten TRIS-NaCl Puffers
+ 50 µl Chromogenes Substrat (S2238 oder S2266)
werden gemischt und die OD/min gemessen.

b) APTT-Verlängerung

    100 µl der PCa-Lösung
+ 100 µl Actin
werden in 100 µl CaCl$_2$ vermischt und die APTT gemessen.

Als Kontrollen werden gereinigtes Protein C und Protein C-Mangelplasma benützt.

European Patent Attorneys – Mandataires en Brevets Européens – zugelassene Vertreter beim Europäischen Patentamt
D-8023 München-Pullach, Wiener Straße 2; Telefon (089) 7 93 30 71; Telex 5 212 147 bros d; Cables: «Patentibus» München

**0182929**

KARL A. BROSE † 1981
Dipl.-Ing.

D. KARL BROSE
Dipl.-Ing., Dipl.-Wirtsch.-Ing.

MICHAEL RESCH
Dipl.-Phys.

American Hospital Supply Corporation
One American Plaza, Evanston, Ill. USA 60201

Ihre Zeichen:
Your ref:

Tag:
Date: 26. November 1984

## Koaktivator zur Aktivierung von Protein C

### A n s p r ü c h e

1. Koaktivator zur Aktivierung von Human-Protein C in Gegenwart von Calciumionen, erhältlich durch folgende Verfahrensschritte:

   a) Ein zerkleinertes frisches menschliches Organ oder eine menschliche Zellkultur wird mit einem TRIS-HCl-Puffer, der Saccharose (0,05 - 0,3 M), Benzamidin (0,1 - 10 mM), Phenyl-methylsulfonyl-fluorid (0,1 - 10 m) und $NaN_3$ (0,01 Gew.-%) enthält, gewaschen und anschließend tiefgefroren.

b) Aus dem tiefgefrorenen Material wird nach dem Auftauen in dem gleichen Puffer wie unter a) ein Homogenat hergestellt und anschließend mit dem gleichen Puffer wie unter a), jedoch mit einem Zusatz von 0,005 bis 5 Gew.-% Detergenz extrahiert.

c) Der Extrakt wird filtriert.

2. Koaktivator nach Anspruch 1, dadurch gekennzeichnet, daß zur Beseitigung einer Thromboplastin-Aktivität der Extrakt nach dem Filtrieren einer Behandlung mit Phospholipase $A_2$ unterworfen wird.

3. Koaktivator nach Anspruch 1 oder 2, dadurch gekennzeichnet, daß als menschliches Organ Placenta verwendet wird.

4. Koaktivator nach einem der vorstehenden Ansprüche, dadurch gekennzeichnet, daß als Detergenz Triton X-100, Tween 20-40-60-80, Cholate, Alkyl-glyco- oder -mannopyranoside, LAPAO, NP-40, Lulepal PX, Lubrol PX, CHAPS oder Sterox verwendet wird.

5. Koaktivator nach einem der vorstehenden Ansprüche, dadurch gekennzeichnet, daß der Extrakt nach dem Filtrieren einer Reinigung unterworfen wird.

6. Koaktivator nach einem der vorstehenden Ansprüche, dadurch gekennzeichnet, daß er zur Stabilisierung einem physikalischen Verfahren unterworfen wird und/oder ihm antimikrobiologische Mittel und/oder $NaN_3$ zugesetzt wird.

7. Verfahren zur Bestimmung des Protein C-Gehalts im menschlichen Plasma, dadurch gekennzeichnet, daß der Probe ein Koaktivator nach einem der vorstehenden Ansprüche zur Umwandlung des Protein C in aktiviertes Protein C zugesetzt wird.

8. Verfahren nach Anspruch 7, dadurch gekennzeichnet, daß der Gehalt des aktivierten Protein C in einem Funktionstest nach einer Gerinnungsmethode oder aufgrund seiner amidolytischen Aktivität bestimmt wird.

9. Verfahren nach Anspruch 8, dadurch gekennzeichnet, daß die amidolytische Aktivität durch Spaltung eines Peptids mit nach der Spaltung analytisch genau und leicht erfaßbarer Indikator-Gruppe erfolgt

10. Diagnostische Reagenz zur Durchführung des Verfahrens nach einem der Ansprüche 7 bis 9, enthaltend Koaktivator nach einem der Ansprüche 1 bis 6.

11. Therapeutisches Mittel, enthaltend Koaktivator nach einem der Ansprüche 1 bis 6 zur Behandlung von Gerinnungsdefekten.

12. Mittel nach Anspruch 11 zur Behandlung von Thrombose.

13. Ausrüstung (kit of parts), enthaltend Koaktivator nach einem der Ansprüche 1 bis 6 sowie Thrombin Inhibitor.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | CHEMICAL ABSTRACTS, Band 101, Nr. 23, 3. Dezember 1984, Seite 246, Nr. 206334z, Columbus, Ohio, US; H.H. SALEM u.a.: "Isolation and characterization of thrombomodulin from human placenta" & J. BIOL. CHEM. 1984, 259(19), 12246-12251 * Zusammenfassung * | 1,3-5, 7,9 | C 12 Q   .1/56 C 12 Q    1/38 G 01 N   33/86 C 12 Q    1/44 A 61 K   35/50 |
| A | BIOLOGICAL ABSTRACTS, Band 77, Nr. 9, 1984, Seite 7137, Nr. 64891, Philadelphia, US; R.B. FRANCIS Jr. u.a.: "A functional assay for protein C in human plasma" & THROMB. RES., 32(6), 605-614, 1983 * Zusammenfassng * | 7,8 | |
| A | US-A-4 214 049   (B.T. AF EKENSTAM u.a.) * Insgesamt * | 7-9 | RECHERCHIERTE SACHGEBIETE (Int. Cl.4) G 01 N C 12 Q A 61 K |
| A | CHEMICAL ABSTRACTS, Band 100, Nr. 19, 7. Mai 1984, Seite 216, Nr. 152824t, Columbus, Ohio, US; N. SALA u.a.: "A functional assay of protein C in human plasma" & BLOOD 1984, 63(3), 671-675 | | |

---      -/-

Der vorliegende Recherchenbericht wurde für alle Patentansprüche erstellt.

| Recherchenort DEN HAAG | Abschlußdatum der Recherche 19-07-1985 | Prüfer GRIFFITH G. |
|---|---|---|

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03.82

Europäisches
Patentamt

EUROPÄISCHER RECHERCHENBERICHT

**0182929**
Nummer der Anmeldung

EP 84 11 4247

Seite 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | CHEMICAL ABSTRACTS, Band 100, Nr. 23, 4. Juni 1984, Seite 383, Nr. 189481e, Columbus, Ohio, US; H.H. SALEM u.a.: "Effects of thrombomodulin and coagulation factor Va-light chain on protein C activation in vitro" & J. CLIN. INVEST. 1984, 73(4), 968-972 | | |
| | --- | | |
| A | CHEMICAL ABSTRACTS, Band 98, Nr. 7, 14. Februar 1983, Seite 476, Nr. 51411r, Columbus, Ohio, US; W.M. CANFIELD u.a.: "Evidence of normal functional levels of activated protein C inhibitor in combined Factor V/VIII deficiency disease" & J. CLIN. INVEST. 1982, 70(6), 1260-1272 | | RECHERCHIERTE SACHGEBIETE (Int. Cl.4) |
| | --- | | |
| A | CHEMICAL ABSTRACTS, Band 97, Nr. 5, 2. August 1982, Seite 375, Nr. 36731f, Columbus, Ohio, US; R.A. MARLAR u.a.: "Mechanism of action of human activated protein C, a thrombin-dependent anticoagulant enzyme" & BLOOD 1982 , 59(5), 1067-1072 | | |
| | ---    -/- | | |

Der vorliegende Recherchenbericht wurde für alle Patentansprüche erstellt.

| Recherchenort | Abschlußdatum der Recherche | Prüfer |
|---|---|---|
| DEN HAAG | 19-07-1985 | GRIFFITH G. |

## EINSCHLÄGIGE DOKUMENTE

Seite 3

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | BIOLOGICAL ABSTRACTS, Band 69, Nr. 4, Seite 2242, Nr. 21158, Philadelphia, US; W. KISIEL: "Human plasma protein C: Isolation, characterization, and mechanism of activation by alpha-thrombin" & J. CLIN. INVEST., 64(3), 761-769, 1979 | | |
| | --- | | |
| A | US-A-3 980 432  (H. TROBISCH u.a.) | | |
| | --- | | |
| A | US-A-3 501 567  (J. CHOAY u.a.) | | |
| | ----- | | |

RECHERCHIERTE
SACHGEBIETE (Int. Cl.4)

Der vorliegende Recherchenbericht wurde für alle Patentansprüche erstellt.

| Recherchenort DEN HAAG | Abschlußdatum der Recherche 19-07-1985 | Prüfer GRIFFITH G. |
|---|---|---|